Europäisches Patentamt

European Patent Office

Office européen des brevets

(11) Veröffentlichungsnummer: **0 281 650 B1**

(12) **EUROPÄISCHE PATENTSCHRIFT**

(45) Veröffentlichungstag der Patentschrift: **04.03.92**

(21) Anmeldenummer: **87103416.1**

(22) Anmeldetag: **10.03.87**

(51) Int. Cl.⁵: **A61M 15/00**

54 **Aerosol-Zerstäuber.**

(43) Veröffentlichungstag der Anmeldung:
**14.09.88 Patentblatt 88/37**

(45) Bekanntmachung des Hinweises auf die
Patenterteilung:
**04.03.92 Patentblatt 92/10**

(84) Benannte Vertragsstaaten:
**AT BE CH DE ES FR GB GR IT LI NL SE**

(56) Entgegenhaltungen:
**EP-A- 0 009 667**
**DE-U- 8 625 936**

(73) Patentinhaber: **Brugger, Stephan,**
**Dipl.-Wirt.-Ing.**
**Etztalstrasse 21**
**W-8137 Berg(DE)**

(72) Erfinder: **Der Erfinder hat auf seine Nennung**
**verzichtet**

(74) Vertreter: **Hoffmann, Klaus, Dr. rer. nat. et al**
**Hoffmann . Eitle & Partner Patentanwälte**
**Postfach 81 04 20 Arabellastrasse 4**
**W-8000 München 81(DE)**

**Beschreibung**

Die Erfindung betrifft einen Aerosol-Zerstäuber zur Inhalationsbehandlung erkrankter Atemwege. Dieser hat ein Unterteil, in das Druckluft eingeleitet wird, einen Medikamentenbecher zur Aufnahme eines Vorrats von wäßriger Medikamenten-Lösung, eine Zerstäubungseinrichtung zur Zerstäubung der Medikamenten-Lösung in einen feinen Aerosol-Nebel mittels der Druckluft, ferner einen Zuluftkamin, durch den Zuluft in den Bereich über der Zerstäubungseinrichtung angesaugt wird, einen Auslaßstutzen für das Gemisch aus Aerosol und Zuluft, und ein Mundstück für den Patienten, welches mit dem Auslaßstutzen in Verbindung steht sowie einem Aerosol-Zerstäuber zur Inhalationsbehandlung erkrankter Atemwege, mit einem Unterteil (1), in das Druckluft eingeleitet wird, einem Medikamentenbecher (2) zur Aufnahme eines Vorrats von wäßriger Medikamenten-Lösung, einer Zerstäubungseinrichtung (3, 4) zur Zerstäubung der Medikamenten-Lösung in einen feinen Aerosol-Nebel mittels der Druckluft, einem Zuluftkamin (6), durch den Zuluft in den Bereich über der Zerstäubungseinrichtung (3, 4) angesaugt wird, einem Auslaßstutzen (13) für das Gemisch aus Aerosol und Zuluft, einem Mundstück für den Patienten, welches mit dem Auslaßstutzen (13) in Verbindung steht, einem zwischen Auslaßstutzen und Mundstück in einem Verteilerstück (14) angeordneten Auslaßventil (19), gekennzeichnet durch ein als Einwegventil ausgebildetes Einlaßventil (7) am Zuluftkamin (6) und einem an das Verteilerstück (14) angeschlossenen Ausatemwiderstand (16). Ein derartiger Aerosol-Zerstäuber ist beispielsweise in der EP-A-0 009 667 beschrieben.

Derartige Aerosol-Zerstäuber werden an einen elektrisch betriebenen Drucklufterzeuger angeschlossen. Über das Mundstück inhaliert der Patient den medikamentenhaltigen feinen Tröpfchennebel. Nur der kleinere Teil der ingesamt eingeatmeten Luft wird dabei von dem Kompressor des Drucklufterzeugers geliefert. Im Bereich über der Zerstäubungseinrichtung wird das mittels der Druckluft aus der flüssigen Medikamenten-Lösung zerstäubte Aerosol mit Zuluft vermischt. Die Anordnung eines Verteilers mit einem Ausatemventil vor dem Mundstück gestattet bei der vorbekannten Konstruktion ein Ausatmen ohne Absetzen des Aerosol-Zerstäubers.

Patienten, welche ihre Atemwegserkrankungen durch Inhalation von Medikamenten-Nebel behandeln müssen, leiden oft gleichzeitig auch an Atemwegverschlüssen. Bei solchen Patienten findet eine weitere Therapieform Anwendung, bei der das Ausatmen der verbrauchten Luft nicht direkt in die Umgebung, sondern über einen genau definierten Strömungswiderstand, eine sog. Stenose, erfolgt.

Das Ausatmen gegen einen gewissen Widerstand baut Sekretionen in den Atemwegen ab und wirkt so Atemwegverschlüssen entgegen. Patienten, die an Atemwegverschlüssen leiden und außerdem auch gezwungen sind, durch Inhalation eines medikamentenhaltigen Aerosols ihre Atemwegerkrankungen zu behandeln, mußten bisher in zwei zeitlich voneinander getrennten Sitzungen behandelt werden. Es war bisher nicht möglich, die beiden Therapieformen der Benutzung eines Inhalators und des Ausatmens gegen einen Atemwiderstand während einer einzigen Behandlungssitzung zu kombinieren.

Aufgabe vorliegender Erfindung ist es daher, einen Aerosol-Zerstäuber zur Inhalationsbehandlung erkrankter Atemwege so weiterzubilden, daß sich mit diesem beide Therapieformen, nämlich sowohl Inhalierung eines medikamentenhaltigen Aerosols als auch Ausatmen gegen einen bestimmten Widerstand, verbinden lassen.

Bei der Lösung dieser Aufgabe wird ausgegangen von einem Aerosol-Zerstäuber der eingangs erwähnten Art. Gelöst wird die Aufgabe dadurch, daß ein als Einwegventil ausgebildetes Einlaßventil am Zuluftkamin vorgesehen ist, ferner ein zwischen Auslaßstutzen und Mundstück angeordnetes Verteilerstück, ein an das Verteilerstück angeschlossener Ausatemwiderstand, gegen den der Patient ausatmet, und schließlich ein als Einwegventil ausgebildetes Auslaßventil zwischen Verteilerstück und Ausatemwiderstand.

Während der Behandlung inhaliert der Patient wie gewohnt den von der Zerstäubungseinrichtung erzeugten Aerosol-Nebel, vermischt mit durch den Zuluftkamin zugeführter Außenluft. Zum Ausatmen wird das Gerät jedoch nicht, wie bisher, vom Mund abgesetzt; der Patient atmet vielmehr über das Mundstück und das Verteilerstück gegen den Ausatemwiderstand aus. Das als Einwegventil ausgebildete Einlaßventil am Zuluftkamin verhindert dabei ein Entweichen von Ausatemluft entgegen der Strömungsrichtung der Zuluft. Das unmittelbar vor dem Ausatemwiderstand angeordnete, ebenfalls als Einwegventil ausgebildete Auslaßventil ist während des Ausatmens geöffnet, verhindert jedoch während der Inhalationsphase ein unerwünschtes Einströmen von Außenluft über den Ausatemwiderstand entgegen der vorgesehenen Strömungsrichtung. Die abwechselnd öffnenden bzw. schließenden Einlaß- und Auslaßventile steuern die Luftströmungen also in der gewünschten Weise. Der mit der Erfindung geschaffene Aerosol-Zerstäuber erlaubt gleichzeitig sowohl eine atemunterstützende Inhalationsbehandlung wie auch eine Atemtherapie mittels künstlichem Ausatemwiderstand während einer einzigen Behandlungssitzung. Die erfindungsgemäße Vorrichtung stellt somit eine Kombination zwischen einem Aerosol-Zerstäuber und einer Atemvorrichtung für Stenose-Behandlung dar.

In vorteilhafter Weiterbildung des Aerosol-Zerstäubers gemäß der Erfindung ist der Ausatemwiderstand auf das Verteilerstück aufsteckbar; dieser läßt sich dann leicht austauschen, um den Strömungswiderstand dem jeweiligen Therapiezweck anzupassen.

Vorzugsweise umfaßt der Ausatemwiderstand eine Lochscheibe, welche das Drosselelement darstellt. Der Ausatemwiderstand kann aber auch mittels einstellbarer Ausblasöffnungen, durch welche die ausgeatmete Luft ins Freie gelangt, gebildet werden. Zweckmäßig ist es, die Lochscheibe austauschbar zu montieren; dabei kann die Lochscheibe beispielsweise von einer aufgeschraubten oder aufgedrückten Kappe in ihrer vorgesehenen Lage gehalten werden.

Von Vorteil ist es ferner, wenn auch das Verteilerstück auf den Auslaßstutzen aufsteckbar ist und ebenso das Mundstück mit dem Verteilerstück bzw. der Zuluftkamin mit dem Oberteil des Aerosol-Zerstäubers steckbar verbunden ist. Das Gerät läßt sich so auf einfache Weise in seine wesentlichen Bestandteile zerlegen und reinigen bzw. desinfizieren. Auch das Einlaßventil am Zuluftkamin sowie das Auslaßventil zwischen Verteilerstück und Ausatemwiderstand sollten zu Reinigungszwecken demontierbar sein.

Bevorzugt wird eine Ausführung, bei der das Auslaßventil und das Einlaßventil aus einem Ringeinsatz und einer gegen die Stirnseite dieses Ringeinsatzes abdichtenden, einseitig angelenkten Klappe besteht. Bevorzugt besteht die Klappe aus flexiblem, insbesondere gummielastischen Material. Die Klappe kann auf einfache Weise mittels einer oder mehrerer, vorzugsweise zwei Stiften auf dem Ringeinsatz befestigt sein. Derartig ausgebildete Einwegventile sind in der Herstellung einfach, dichten gut ab und zeichnen sich durch eine lange Lebensdauer aus.

Besonders vorteilhaft ist die Ausführung des Verteilerstücks als T-Stück. Der Ausatemwiderstand ist dann an dessen mittlerem Stutzen angeschlossen. Diese geometrische Ausgestaltung zeichnet sich wegen der geradlinigen Verbindung zwischen Auslaßstutzen und Mundstück durch einen besonders geringen Strömungswiderstand beim Einatmen aus, so daß das medikamentenhaltige Aerosol weitgehend ungehindert in den Rachen des Patienten gelangt.

Am Verteilerstück kann ferner eine verschließbare Öffnung vorgesehen sein, an welcher eine Druckmeßvorrichtung anschließbar ist. Der von den Lungen des Patienten aufzubringende Druck beim Ausatmen kann so exakt kontrolliert werden.

Ein zusätzliches Filter, das vor oder nach dem Ausatemwiderstand angeordnet sein kann, verhindert eine oft unerwünschte Kontaminierung der Umgebungsluft mit Medikamentenresten, welche noch in der Ausatemluft enthalten sind.

Ein Ausführungsbeispiel der Erfindung wird nachstehend anhand der beigefügten Zeichnungen näher erläutert. Es zeigen:

Fig. 1 Einen Aerosol-Zerstäuber, mit aufsteckbarem T-förmigen Verteilerstück, in einem Schnittbild;

Fig. 2 das in dem Aerosol-Zerstäuber gemäß Fig. 1 eingesetzte Auslaßventil, in einer vergrößerten perspektivischen Darstellung.

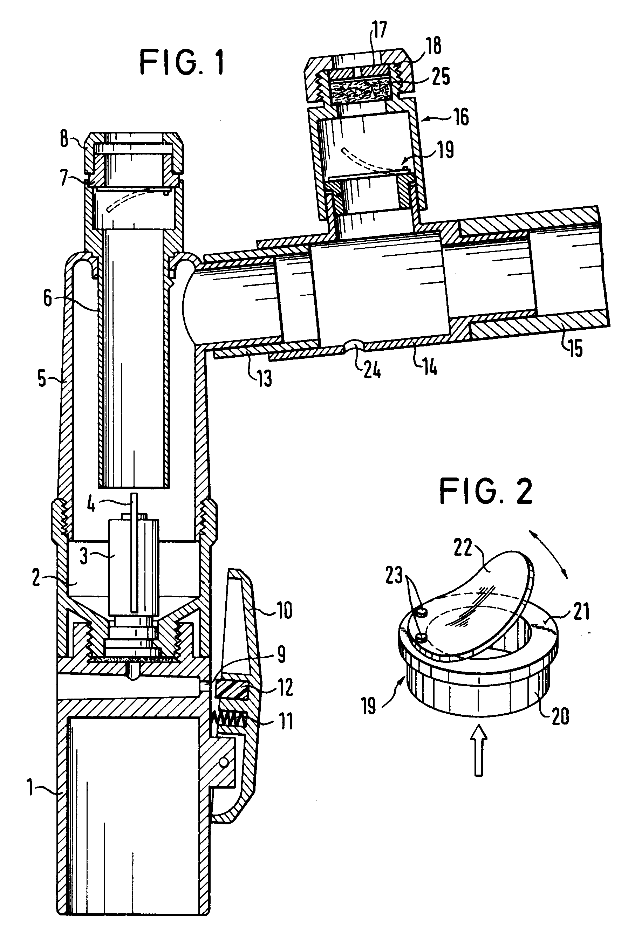

Der in Fig. 1 dargestellte Aerosol-Zerstäuber weist ein rohrförmiges Unterteil 1 auf, welches mit einem (nicht dargestellten) elektrisch betriebenen Drucklufterzeuger in Verbindung steht. Im Unterteil 1 ist ein Medikamentenbecher 2 ausgebildet, welcher einen Vorrat von wäßriger Medikamenten-Lösung aufnehmen kann. Ein koaxial in der Mitte des Medikamentenbechers 2 angeordneter Düsenkörper 3 und ein auf diesem aufgesetztes Luftstromsteuer 4 bilden gemeinsam eine Zerstäubungseinrichtung, welche die flüssige Medikamenten-Lösung mittels der Druckluft, die durch Bohrungen innerhalb des Düsenkörpers 3 geblasen wird, in einen feinen Aerosol-Nebel zerstäubt. Auf das Unterteil 1 ist ein ebenfalls rohrförmiges Oberteil 5 aufgesetzt. Dieses Oberteil 5 weist oben eine runde Öffnung auf, in welche ein hohlzylindrischer Zuluftkamin 6 eingesetzt ist. Mit seinem unteren Ende ragt der Zuluftkamin 6 bis unmittelbar über das Luftstromsteuer 4, so daß Außenluft von oben angesaugt und in den Bereich unmittelbar über dem Düsenkörper 3 geleitet wird. Die eingeleitete Zuluft vermischt sich mit dem medikamentenhaltigen Aerosol-Nebel. An seinem oberen Ende ist in den Zuluftkamin 6 ein Einlaßventil 7 eingesetzt, welches als Einwegventil ausgebildet ist. Das Einlaßventil 7 ist demontierbar und wird von einer auf das obere Ende des Zuluftkamins 6 aufgeschraubten Kappe 8 gehalten.

Am Unterteil 1 ist ferner eine kleine Ventilöffnung 9 vorgesehen, durch welche die eingeleitete Druckluft in die Umgebung entweichen kann, bevor sie in den Düsenkörper einströmt. Die Ventilöffnung 9 kann durch Betätigung einer Ventiltaste 10 verschlossen werden. Hierzu muß die Ventiltaste 10 entgegen der Wirkung einer Feder 11 in Richtung des Unterteils 1 verschwenkt werden, wodurch sich eine elastische Tastendichtung 12 auf die Ventilöffnung 9 preßt und diese luftdicht verschließt. Mittels der Ventiltaste 10 kann somit die Aerosol-Erzeugung während des Ausatmens unterbrochen werden, ohne daß hierzu der elektrische Drucklufterzeuger abgeschaltet werden müßte. Wird die Ventiltaste 10 gedrückt, setzt sofort die Vernebelung der Medikamenten-Lösung ein.

Am Oberteil 5 ist seitlich ein im Querschnitt runder Auslaßstutzen 13 angeformt, über den das Gemisch aus Zuluft und medikamentenhaltigem Aerosol austritt. An den Auslaßstutzen 13 schließt sich ein T-förmiges Verteilerstück 14 an. Ein Mundstück 15 für den Patienten ist auf das Verteilerstück 14 aufgesteckt. An den mittleren Stutzen des Verteilerstücks 14 ist ein Ausatemwiderstand 16 angeschlossen. Als Drosselelement enthält der Ausatemwiderstand 16 eine Lochscheibe 17, welche mittels einer aufgeschraubten Kappe 18 in ihrer vorgesehenen Lage quer zur Strömungsrichtung gehalten ist. Zwischen dem Verteilerstück 14 und dem Ausatemwiderstand 16 ist ein Auslaßventil 19 angeordnet, welches wiederum als Einwegventil ausgebildet ist.

Zur Inhalation von medikamentenhaltigem Aerosol atmet der Patient durch das Mundstück 15 ein, wobei er gleichzeitig die Ventiltaste 10 drückt. Das Auslaßventil 19 vor dem Ausatemwiderstand 16 ist dabei geschlossen, während das Einlaßventil 7 geöffnet ist, so daß Außenluft ausschließlich durch den Zuluftkamin 6 einströmen kann. Atmet der Patient anschließend aus, so kehrt sich die Strömungsrichtung innerhalb des Verteilerstücks 14 um. Das Auslaßventil 19 vor dem Ausatemwiderstand 16 öffnet, während das Einlaßventil 7 am Zuluftkamin 6 infolge des einwirkenden Druckes schließt. Die ausgeatmete Luft kann jetzt nur durch das Loch in der Lochscheibe 17 des Ausatemwiderstands 16 ins Freie gelangen. Die Aerosol-Vernebelung kann solange durch Loslassen der Ventiltaste 10 unterbrochen werden.

Am Verteilerstück 14 ist eine verschließbare Öffnung 24 zum Anschluß einer (nicht dargestellten) Druckmeßvorrichtung vorgesehen. Unmittelbar vor der Lochscheibe 17 des Ausatemwiderstands 16 ist ein hochporöses Filter 25 angeordnet, das das in der Ausatemluft enthaltene restliche Medikament zurückhält.

Fig. 2 zeigt das Auslaßventil 19. Es besteht aus einem Ringeinsatz 20, auf dessen Stirnseite 21 eine Klappe 22 einseitig angelenkt ist. Die Klappe 22 besteht aus gummielastischem Material und ist mittels zweier Stifte 23 auf der Stirnseite 21 des Ringeinsatzes 20 befestigt. Der Ringeinsatz 20 selbst sowie die Stifte 23 bestehen aus Kunststoff. In geschlossenem Zustand dichtet die Klappe 22 gegenüber der ebenen Stirnseite 21 ab. Bei Durchströmung in Pfeilrichtung von unten nach oben hebt die flexible Klappe 22 in die gezeigte Stellung ab, so daß das Ventil öffnet. - Das Einlaßventil 7 am Zuluftkamin 6 ist von derselben Art wie das beschriebene und in Fig. 2 dargestellte Auslaßventil 19.

Alle wesentlichen Einzelteile des Aerosol-Zerstäubers sind klemmend oder schraubbar miteinander verbunden. So sind Verteilerstück 14, Ausatemwiderstand 16, Mundstück 15 und Auslaßstutzen 13 jeweils ineinander gesteckt und lassen sich zu Reinigungszwecken leicht voneinander abziehen.

Verzeichnis der verwendeten Bezugsziffern

| | |
|---|---|
| 1 | Unterteil |
| 2 | Medikamentenbecher |
| 3 | Düsenkörper |
| 4 | Luftstromsteuer |
| 5 | Oberteil |
| 6 | Zuluftkamin |
| 7 | Einlaßventil (an 6) |
| 8 | Kappe (auf 6) |
| 9 | Ventilöffnung (in 1) |
| 10 | Ventiltaste |
| 11 | Feder (an 10) |
| 12 | Tastendichtung (an 10) |
| 13 | Auslaßstutzen |
| 14 | Verteilerstück |
| 15 | Mundstück |
| 16 | Ausatemwiderstand |
| 17 | Lochscheibe (von 16) |
| 18 | Kappe (auf 16) |
| 19 | Auslaßventil (zwischen 14 und 16) |
| 20 | Ringeinsatz (von 19) |
| 21 | Stirnseite (von 20) |
| 22 | Klappe (von 19) |
| 23 | Stifte |
| 24 | Öffnung (in 14) |
| 25 | Filter (in 14) |

**Patentansprüche**

1. Aerosol-Zerstäuber zur Inhalationsbehandlung erkrankter Atemwege, mit
   - einem Unterteil (1), in das Druckluft eingeleitet wird,
   - einem Medikamentenbecher (2) zur Aufnahme eines Vorrats von wäßriger Medikamenten-Lösung,
   - einer Zerstäubungseinrichtung (3, 4) zur Zerstäubung der Medikamenten-Lösung in einen feinen Aerosol-Nebel mittels der Druckluft,
   - einem Zuluftkamin (6), durch den Zuluft in den Bereich über der Zerstäubungseinrichtung (3, 4) angesaugt wird,
   - einem Auslaßstutzen (13) für das Gemisch aus Aerosol und Zuluft,
   - einem Mundstück für den Patienten, welches mit dem Auslaßstutzen (13) in Verbindung steht,
   - einem zwischen Auslaßstutzen und Mundstück in einem Verteilerstück (14) angeordneten Auslaßventil (19),
   **gekennzeichnet durch**
   - ein als Einwegventil ausgebildetes Einlaßventil (7) am Zuluftkamin (6) und

- einem an das Verteilerstück (14) angeschlossenen Ausatemwiderstand (16).

2. Aerosol-Zerstäuber nach Anspruch 1, dadurch gekennzeichnet, daß der Ausatemwiderstand (16) auf das Verteilerstück (14) aufsteckbar ist.

3. Aerosol-Zerstäuber nach Anspruch 1 oder 2, dadurch gekennzeichnet, daß der Ausatemwiderstand (16) eine Lochscheibe (17) als Drosselelement umfaßt.

4. Aerosol-Zerstäuber nach Anspruch 3, dadurch gekennzeichnet, daß die Lochscheibe (17) austauschbar ist.

5. Aerosol-Zerstäuber nach Anspruch 4, dadurch gekennzeichnet, daß die Lochscheibe (17) von einer aufgeschraubten oder aufgedrückten Kappe (18) gehalten wird.

6. Aerosol-Zerstäuber nach einem der Ansprüche 1 bis 5, dadurch gekennzeichnet, daß das Verteilerstück (14) auf den Auslaßstutzen (13) aufsteckbar ist.

7. Aerosol-Zerstäuber nach einem der Ansprüche 1 bis 6, dadurch gekennzeichnet, daß das Mundstück (15) auf das Verteilerstück (14) aufsteckbar ist.

8. Aerosol-Zerstäuber nach einem der Ansprüche 1 bis 7, dadurch gekennzeichnet, daß der Zuluftkamin (6) abnehmbar ist.

9. Aerosol-Zerstäuber nach einem der Ansprüche 1 bis 8, dadurch gekennzeichnet, daß das Einlaßventil (7) und/oder das Auslaßventil (19) demontierbar ist.

10. Aerosol-Zerstäuber nach einem der Ansprüche 1 bis 9, dadurch gekennzeichnet, daß das Auslaßventil (19) und/oder das Einlaßventil (7) aus einem Ringeinsatz (20) und einer gegen die Stirnseite (21) dieses Ringeinsatzes abdichtenden, einseitig angelenkten Klappe (22) bestehen.

11. Aerosol-Zerstäuber nach Anspruche 10, dadurch gekennzeichnet, daß die Klappe (22) aus flexiblem, insbesondere gummielastischen Material besteht.

12. Aerosol-Zerstäuber nach Anspruche 11, dadurch gekennzeichnet, daß die Klappe (22) mittels einem oder mehreren, insbesondere zwei Stiften (23) auf dem Ringeinsatz (20) befestigt ist.

13. Aerosol-Zerstäuber nach einem der Ansprüche 1 bis 12, dadurch gekennzeichnet, daß das Verteilerstück (14) als T-Stück ausgeführt ist, wobei der Ausatemwiderstand (16) am mittleren Stutzen angeschlossen ist.

14. Aerosol-Zerstäuber nach einem der Ansprüche 1 bis 13, dadurch gekennzeichnet, daß am Verteilerstück (14) eine verschließbare Öffnung (24) für den Anschluß einer Druckmeßvorrichtung vorgesehen ist.

15. Aerosol-Zerstäuber nach einem der Ansprüche 1 bis 14, dadurch gekennzeichnet, daß vor oder nach dem Ausatemwiderstand (16) ein Filter (25) für die Ausatemluft vorgesehen ist.

## Claims

1. An aerosol atomizer for the inhalation treatment of diseased respiratory ducts comprising
   - a lower part (1) into which compressed air is introduced,
   - a medicament container (2) for the accommodation of a supply of aqueous medicament solution,
   - an atomizing device (3, 4) for the atomization of the medicament solution into a fine aerosol mist by means of the compressed air,
   - an air inlet pipe (6) through which inlet air is sucked into the region above the atomizing device (3, 4),
   - an outlet pipe (13) for the mixture of aerosol and inlet air,
   - a mouth-piece for the patient which is connected to the outlet pipe (13),
   - an outlet valve (19) arranged between the outlet pipe and the mouth-piece in a distributor component (14),
   characterised by
   - an inlet valve (7) which is designed as a one-way valve and is arranged in the air inlet pipe (6) and
   - an exhalation resistance (16) connected to the distributor component (14).

2. An aerosol atomizer as claimed in Claim 1, characterised in that the exhalation resistance (16) is attachable to the distributor component (14).

3. An aerosol atomizer as claimed in Claim 1 or 2, characterised in that the exhalation resistance (16) comprises an apertured plate (17) as throttling element.

4. An aerosol atomizer as claimed in Claim 3, characterised in that the apertured plate (17) is exchangeable.

5. An aerosol atomizer as claimed in Claim 4, characterised in that the apertured plate (17) is retained by a screw-on or press-on cap (18).

6. An aerosol atomizer as claimed in one of Claims 1 to 5, characterised in that the distributor component (14) is attachable to the outlet pipe (13).

7. An aerosol atomizer as claimed in one of Claims 1 to 6, characterised in that the mouthpiece (15) is attachable to the distributor component (14).

8. An aerosol atomizer as claimed in one of Claims 1 to 7, characterised in that the air inlet pipe (6) is removable.

9. An aerosol atomizer as claimed in one of Claims 1 to 8, characterised in that the inlet valve (7) and/or the outlet valve (19) is/are dismountable.

10. An aerosol atomizer as claimed in one of Claims 1 to 9, characterised in that the outlet valve (19) and/or the inlet valve (7) comprise an annular insert (20) and a flap (22) which provides a seal vis-a-vis the end side (21) of said annular insert and which is hinged on one side.

11. An aerosol atomizer as claimed in Claim 10, characterised in that the flap (22) is composed of flexible, in particular rubber-elastic material.

12. An aerosol atomizer as claimed in Claim 11, characterised in that the flap (22) is attached to the annular insert (20) by means of one or more, in particular two, pins (23).

13. An aerosol atomizer as claimed in one of Claims 1 to 12, characterised in that the distributor component (14) is designed as a T-piece, where the exhalation resistance (16) is connected to the central pipe of said T-piece.

14. An aerosol atomizer as claimed in one of Claims 1 to 13, characterised in that the distributor component (14) is provided with a closable opening (24) for the connection of a pressure measuring device.

15. An aerosol atomizer as claimed in one of Claims 1 to 14, characterised in that a filter (25) for the exhaled air is arranged in advance of or following the exhalation resistance (16).

**Revendications**

1. Pulvérisateur d'aérosol, pour le traitement par inhalation de maladies des voies respiratoires, comportant :
   - une partie inférieure (1), dans laquelle de l'air comprimé est introduit,
   - un bécher à médicament (2), pour recevoir une réserve de solution médicamenteuse aqueuse,
   - un dispositif de pulvérisation (3,4), pour pulvériser la solution médicamenteuse en un fin brouillard aérosol, au moyen de l'air comprimé,
   - une cheminée d'air d'alimentation (6), par laquelle l'air d'alimentation est aspiré dans la zone située au-dessus du dispositif de pulvérisation (3,4),
   - une tubulure d'évacuation (13) pour le mélange composé d'aérosol et d'air d'alimentation,
   - une pièce d'embouchure pour le patient, qui est reliée à la tubulure d'évacuation (13),
   - une soupape d'évacuation (19), disposée entre la tubulure d'évacuation et la pièce d'embouchure, dans une pièce distributrice (14),
   caractérisé par
   - une soupape d'admission (7) réalisée sous forme de soupape unidirectionnelle, sur la cheminée d'air d'alimentation (6), et
   - une résistance d'exhalaison (16) raccordée à la pièce distributrice (14).

2. Pulvérisateur d'aérosol selon la revendication 1, caractérisé en ce que la résistance d'exhalaison (16) est enfichable sur la pièce distributrice (14).

3. Pulvérisateur d'aérosol selon la revendication 1 ou 2, caractérisé en ce que la résistance d'exhalaison (16) comprend un disque percé (17) servant d'élément d'étranglement.

4. Pulvérisateur d'aérosol selon la revendication 3, caractérisé en ce que le disque percé (17) est interchangeable.

5. Pulvérisateur d'aérosol selon la revendication 4, caractérisé en ce que le disque percé (17) est maintenu par un capuchon (18) vissé ou pressé.

6. Pulvérisateur d'aérosol selon l'une des revendications 1 à 5, caractérisé en ce que la pièce distributrice (14) est enfichable sur la tubulure d'évacuation (13).

7. Pulvérisateur d'aérosol selon l'une des revendications 1 à 6, caractérisé en ce que la pièce d'embouchure (15) est enfichable sur la pièce distributrice (14).

8. Pulvérisateur d'aérosol selon l'une des revendications 1 à 7, caractérisé en ce que la cheminée d'air d'alimentation (6) est amovible.

9. Pulvérisateur d'aérosol selon l'une des revendications 1 à 8, caractérisé en ce que la soupape d'admission (7) et/ou la soupape d'évacuation (19) est démontable.

10. Pulvérisateur d'aérosol selon l'une des revendications 1 à 9, caractérisé en ce que la soupape d'évacuation (19) et/ou la soupape d'admission (7) se composent d'une garniture annulaire (20) et d'un clapet (22) articulé à débattement d'un côté; assurant un isolation étanche face à la face frontale (21) de cette garniture annulaire.

11. Pulvérisateur d'aérosol selon la revendication 10, caractérisé en ce que le clapet (22) est en un matériau flexible, en particulier ayant l'élasticité du caoutchouc.

12. Pulvérisateur d'aérosol selon la revendication 11, caractérisé en ce que le clapet (22) est fixé sur la garniture annulaire (20) au moyen d'une ou plusieurs goupilles (23), en particulier deux goupilles.

13. Pulvérisateur d'aérosol selon l'une des revendications 1 à 12, caractérisé en ce que la pièce distributrice (14) est réalisée sous forme de pièce en T, la résistance d'exhalaison (16) étant raccordée à la tubulure centrale.

14. Pulvérisateur d'aérosol selon l'une des revendications 1 à 13, caractérisé en ce qu'une ouverture (24) obturable est prévue sur la pièce distributrice (14), pour le raccordement d'un dispositif de mesure de pression.

15. Pulvérisateur d'aérosol selon l'une des revendications 1 à 14, caractérisé en ce qu'un filtre (25) pour l'air d'exhalaison est prévu, en amont ou en aval de la résistance d'exhalaison (16).

# FIG. 1

# FIG. 2